# EUROPEAN PATENT APPLICATION

(11) **EP 2 883 963 A1**
(43) Date of publication of application: **17.06.2015**
(21) Application number: 13197470.1
(22) Date of filing: 16.12.2013
(51) Int. Cl.: C12Q 1/68

(54) **Method for generating of oligonucleotide arrays using in situ block synthesis approach**

(71) Applicant: Alacris Theranostics GmbH, 14195 Berlin (DE); Max-Planck-Gesellschaft zur Förderung der Wissenschaften e.V., 80539 München (DE)
(72) Inventor: Lehrach, Hans, 14195 Berlin (DE); Borodina, Tatjana, 14195 Berlin (DE); Rykalina, Vera, 14195 Berlin (DE)
(74) Representative: CH Kilger Anwaltspartnerschaft mbB

(57) **Abstract**

The idea of this invention is to prepare ordered oligonucleotides arrays from two or more presynthesized shorter parts - block-synthesis approach. The parts are linked together enzymatically to form a full-length oligonucleotide of a desired sequence. Such an approach allows splitting the oligonucleotide sequences into common and unique parts. It gives the possibility to place the functional group on a common part and to minimize the length of the unique parts. Method of the invention allows combinatorial synthesis of position-specific regions. Using combinatorial approach, position-specific regions are generated by linking two or more unique oligonucleotides, so that just few said unique oligonucleotides give rise to a large variety of codes, for example, 10 unique oligonucleotides linked pairwise can produce 100 position-specific regions.

In comparison to preparation of oligonucleotide arrays by spotting of full-length sequences, suggested approach is more cost-efficient, allows flexibility in generating position-specific unique sequences and is less prone to oligonucleotide length restrictions.

In comparison to *in situ* synthesis of oligonucleotides from nucleotides, current invention allows cost-efficient solution for synthesis of oligonucleotides with free 3' ends.

Important application of the current invention is preparation of two-dimensional oligonucleotide arrays for preparation of sequencing libraries from 2D distributed NA molecules. Oligonucleotides on such arrays need to have position-specific sequences and free 3' ends for further enzymatic reactions.

## Description

### Field of the invention

The present invention is in the field of molecular biology, more precisely in the field of oligonucleotides synthesis, more precisely in the field of preparation of oligonucleotide arrays.

### Background

2D oligonucleotide arrays are widely used in molecular biology.

Oligonucleotide arrays may be prepared by in situ synthesis on the surface from individual nucleotides (Agilent, Affymetrix). During such synthesis oligonucleotides are synthesized in 3' to 5' direction, so that their 3' ends are attached to the surface. Synthesis in other direction (5' to 3') is also possible, but is much more difficult and expensive and currently is not available commercially.

For many applications free 3' ends of the oligonucleotides on the array are required. The current solution is to immobilize full-length pre-synthesized oligonucleotides. However when large number of types of oligonucleotides have to be immobilized this is very expensive. One of the parameters contributing to the high costs is functional groups required for immobilization. Usually it would at least double the price of oligonucleotides. Besides, full-length oligonucleotides have often a considerable length, which also contributes to the price.

In the present invention we suggest an approach of preparation of oligonucleotide arrays using shorter conventionally synthesized oligonucleotides. From such shorter "blocks" the full-length oligonucleotides are built up. We describe several realizations of this approach using enzymatic reactions to combine those "blocks".

### Brief description of the invention

The invention relates to a method for generating a two-dimensional oligonucleotide array comprising a method for generating a two-dimensional oligonucleotide array comprising
a) providing a solid support, which allows immobilization of the first set of oligonucleotides;
b) providing a first set of synthetic oligonucleotides comprising a functional group, which allows immobilization on said solid support, optionally one or more position-labeling (sub-)sequences, optionally sequences used for the later array applications, optionally appropriate junction sequences allowing the linking of the different oligonucleotides either before or after spotting;
c) immobilizing said first set of synthetic oligonucleotides on the solid support
d) attaching to said immobilized oligonucleotides at least one additional set of oligonucleotides in an ordered manner, wherein said other sets of oligonucleotides comprise one or more position labeling (sub-)sequences, optionally appropriate junction sequences allowing the linking of the to the said first set of sequences or other sets and optionally an additional capture sequence.

The idea of this invention is to prepare ordered oligonucleotide arrays from two or more pre-synthesized shorter parts - block-synthesis approach. The parts are linked together enzymatically to form a full-length oligonucleotide of a desired sequence. Such approach allows splitting the oligonucleotide sequences into common and unique parts. It gives the possibility to place the functional group on a common part and to minimize the length of the unique parts. Method of the invention allows combinatorial synthesis of position-specific regions. Using combinatorial approach, position-specific regions are generated by linking two or more unique oligonucleotides, so that just few said unique oligonucleotides give rise to a large variety of codes, for example, 10 unique oligonucleotides linked pairwise can produce 100 position-specific regions.

In comparison to preparation of oligonucleotide arrays by spotting of full-length sequences, suggested approach is more cost-efficient, allows flexibility in generating position-specific unique sequences and is less prone to oligonucleotide length restrictions.

In comparison to *in situ* synthesis of oligonucleotides from nucleotides, current invention allows cost-efficient solution for synthesis of oligonucleotides with free 3' ends.

Important application of the current invention is preparation of two-dimensional oligonucleotide arrays for construction of sequencing libraries from 2D distributed nucleic acid molecules. Oligonucleotides on such arrays need to have position-specific sequences and free 3' ends for further enzymatic reactions.

In 2005 we patented Ligation-based synthesis of oligonucleotides with block structure (EP 1 616 008 A2), which suggested synthesizing long oligonucleotides from shorter oligonucleotides in solution. Current application partly uses the same idea in application of oligonucleotide array preparation.

### Definitions

Oligonucleotides may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981), which is hereby incorporated by reference. One method for synthesizing oligonucleotides on a modified solid support is described in U.S. Pat. No. 4,458,006, which is hereby incorporated by reference. It is also possible to use an oligonucleotide which has been isolated from a biological source (such as a restriction endonuclease digest).

Functional group: an oligonucleotide modification which allows specific binding of oligonucleotides to the surface.

Position labelling or position specific region in an oligonucleotide which should be specific for certain positions on the array.

Within the context of the invention the term junction sequence refers to a defined nucleic acid sequence on one oligonucleotide and to a homologous or complementing sequence on a second oligonucleotide, allowing hybridization of the two oligonucleotides.

Besides position-specific and junction region, oligonucleotides may contain sequences required for later applications of the array (for example, for hybridisation to nucleic acid molecules applied to the array), and other sequences which might have or might not have any particular purpose. For example, there may be regions just for increasing the length of the oligonucleotide, to provide certain melting temperature, to provide binding sites for certain proteins, etc.

### Detailed Description of the Invention

The idea of this invention is to prepare oligonucleotides on the arrays from pre-synthesized shorter parts - block-synthesis approach. These parts are linked together enzymatically to form a full-length oligonucleotide of desired sequence. Such an approach makes the use of splitting the oligonucleotide sequences into common and unique parts. It allows to:
- place the functional group on a common part,
- keep minimal length of unique parts.
- in case a position-specific code is required for future applications, this approach allows to use minimal number of unique parts for synthesis of a desired number of codes (combinatorial code synthesis)
- this approach avoids any oligonucleotide length restrictions
- both directions of oligonucleotides (3'→5' or 5'→3') on the array are possible.

Oligonucleotides on array may be covalently or non-covalently bound to the array surface.

Examples for non-covalent binding methods for nucleotides are: Ni-NTA interaction, maltose-maltose-binding-protein interactions, biotin-streptavidin interaction.

Examples of covalent binding methods are: binding of thiol- or amino- modified oligonucleotides to the epoxy-, carboxy-, or aldehyde- modified glass surface, copolymerization of the acrydite-modified oligonucleotides with the acrylamide.

Shorter oligonucleotides from which full-length oligonucleotides are synthesized on the surface of the array may be synthesized using solid-phase synthesis or any other method the synthesis of synthetic oligonucleotides.

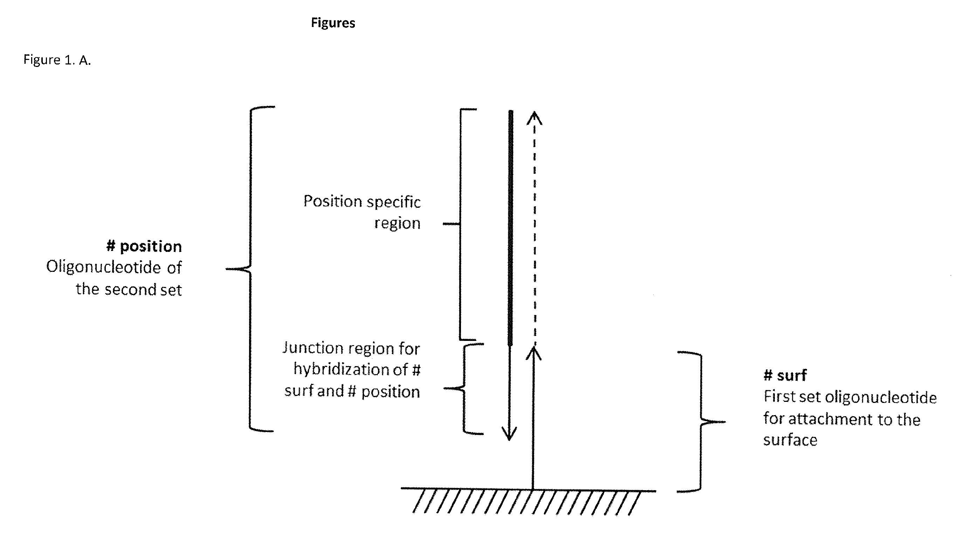

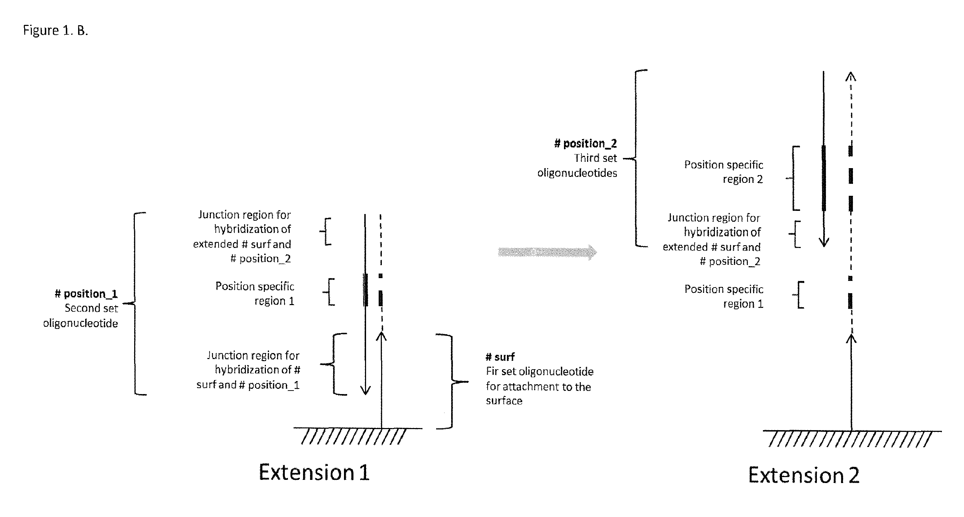

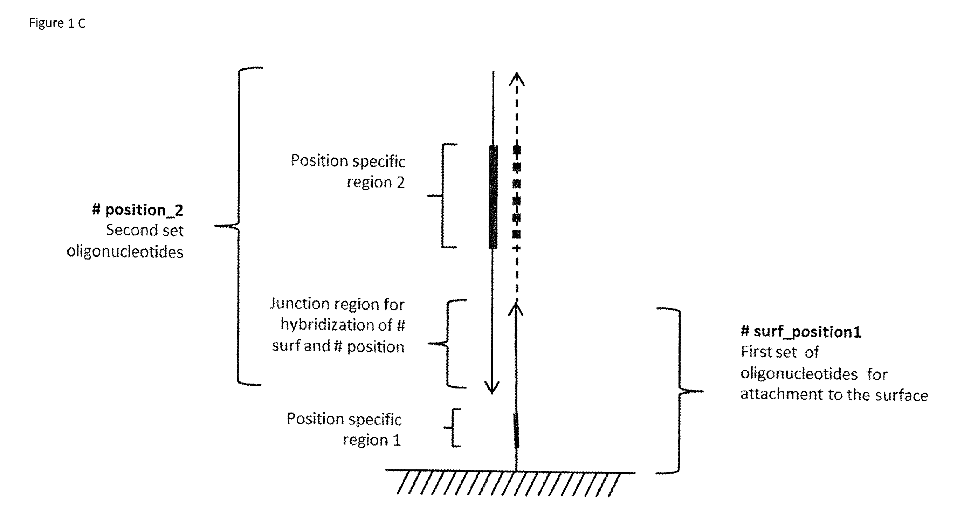

Figures 1 to 3 show different alternative variations of preparation of full-length oligonucleotides on the surface according to the present invention. Full-length oligonucleotides should be prepared from at least two shorter oligonucleotides, wherein at least one should have position-specific region within its sequence.

Oligonucleotides are spotted to a certain position on a microarray one after another. There may be washing of previous set before adding the next one, or there may be not. If more than two sets of oligonucleotides are used for preparation of full-length oligonucleotide on the surface, enzymatic addition of oligonucleotides may be performed for each set separately, or for all together.

In a preferred embodiment the oligonucleotides in each of the two sets have a length of less than 200 nucleotides, preferably less than 100 nucleotides, more preferably less than 75 nucleotides, even preferably less than 50 nucleotides, most preferably less than 40 nucleotides.

The length of the position specific sequence in either set of oligonucleotides is directly dependent on the amount of "coordinates" necessary on the 2D dimensional array. A label sequence with a length of 10 nucleotides can provide a possible differentiation of 1048576 positions, if it therefore two label sequences, each comprising 10 nucleotides, would be combined it would allow a possible differentiation of about 1.1 x10¹² spatial positions, which would be suitable to differentiate single oligonucleotides on said array. If only distinct regions on said array should be differentiated a shorter label sequence might be suitable.

The defined junction sequence on both sets of oligonucleotides may or may not be present. If present, the two sets of nucleotides should be able to hybridize to each other over said junction region. Hence the junction region on the second set of oligonucleotides should preferably represent a complement of the region of the first set of oligonucleotides. The length of said region should be sufficient to allow stable binding of the two regions.

In one embodiment of the invention the junction sequence of the oligonucleotides of the first and/or second set has a length of 20 or less nucleotides, in a preferred embodiment the junction sequence has length of 15 or less nucleotides and in a more preferred embodiment the junction sequence has a length of 10 or less nucleotides. In the most preferred embodiment the junction sequence of the first and/or second set of oligonucleotides has a length of 10, 9, 8, 7, 6, 5 or 4 nucleotides.

Oligonucleotides may additionally comprise a capture sequence or a reverse complement thereof for capturing of nucleic acid molecules applied to the array. The capture sequence might be used to limit the number of target molecules. Any sequence may be suitable as capture sequence, non-limiting examples are: sequences of short-tandem repeats, known single-nucleotide polymorphisms or simply a repetitive sequence, random sequence, locus-specific sequence. Capture sequences may be different within one set of oligonucleotides.

The oligonucleotides of one or both of the two subsequent sets of oligonucleotides might include other sequences, e.g. another label sequence or spacer sequences. Additionally, the oligonucleotides of either set might comprise a restriction site or form a restriction site, which is present when the sequences of the oligonucleotides of both sets are fused.
Preferably according to one embodiment of this invention the two sets of oligonucleotides are single-stranded oligonucleotides. In another embodiment of the invention at least one set of oligonucleotides comprises single-stranded oligonucleotides. In yet another embodiment at least one set of oligonucleotides comprises double-stranded oligonucleotides.

To form the solid support it is necessary to connect the sequences of the two or more sets of oligonucleotides. There are several possible ways to do, non-limiting examples include: extension reaction, ligation, recombination, or a combination thereof.

Preferably the connection of the sequences is performed in a way that the oligonucleotides on the array are uniquely identifiable and the combinations of position specific sequences allow an exact identification of the position of the oligonucleotide or a group of oligonucleotides on the two dimensional array.

The array is preferably created on a solid support. The solid support may be made of any suitable material. Preferred but non-limiting examples include the following materials for the solid support: glass, plastic, metal, paper, or a membrane. In a preferred embodiment of the invention the material of the solid support is glass.

The solid support might be coated to allow binding of the oligonucleotides. The coating may be of any material as suitable. In one embodiment the coating is a gel. In a preferred embodiment the solid support is coated with substances to allow the immobilization of the first set of oligonucleotides, preferably by covalent or non-covalent binding. In a more preferred embodiment the coating of the solid support allows non-covalent immobilization of the first set of oligonucleotides. In another embodiment the coating comprises multiple components.

In one embodiment of the invention the first set of oligonucleotides is immobilized on the solid support in an ordered manner. Preferably the immobilization of the different position specific sequences is done in an ordered manner in a way that the first label could act as a coordinate in a coordinate system and would already allow a broad distinction of the regions on the solid support (see Figure 5a).

In another embodiment of the invention, the solid support is split in multiple parts, which could later be assembled together (see Figure 5b) and of the oligonucleotides of the first set only oligonucleotides with a distinct position specific sequence are immobilized on particular parts of the solid support. There may be a plurality of parts comprising the same label.

The oligonucleotides may be immobilized by any suitable way. Preferably the immobilization is covalent. It is important that the immobilization is stable under conditions, which would cleave double stranded DNA. If double stranded oligonucleotides are used and immobilized, preferably only one strand of the double stranded oligonucleotide is immobilized.

Oligonucleotides of the first set, which had not been immobilized, are preferably removed. The person skilled in the art knows suitable methods to remove non-immobilized nucleotides. Preferably the unbound oligonucleotides are removed by multiple washing steps.

To create a 2D-array according to the present invention it is now necessary to add a second location information, present in the position-specific sequence of the second set of oligonucleotides. In one embodiment of the invention the second set of oligonucleotides is added and the sequence of the second set is added to the immobilized oligonucleotides, thus creating elongated oligonucleotides comprising at least two label sequences. In a preferred embodiment of the invention the sequences of the second set of oligonucleotides are added in a manner, that each single oligonucleotide has a unique combination of labels or only a group of oligonucleotides in close proximity has the same label and each group label is unique. In a preferred embodiment each oligonucleotide has a unique label.

The person skilled in the art readily knows suitable methods to transfer the sequence information onto the first set of oligonucleotides immobilized on the solid support. Non-limiting examples of potential methods include: elongation by a polymerase, ligation, recombination or a combination thereof.

In a preferred embodiment of the invention the subsequent sets of oligonucleotides consist of single-stranded oligonucleotides according to oligonucleotides #surf and #position (e.g. Figure 1A). In this particular embodiment and any other embodiment, wherein the oligonucleotides of the subsequent sets comprise a complementary region, the sequence information may be transferred by primer extension. In a preferred embodiment the respective other strand serves as template and the amplification is done using Klenow polymerase, creating a double-stranded oligonucleotide (Figure 1A).

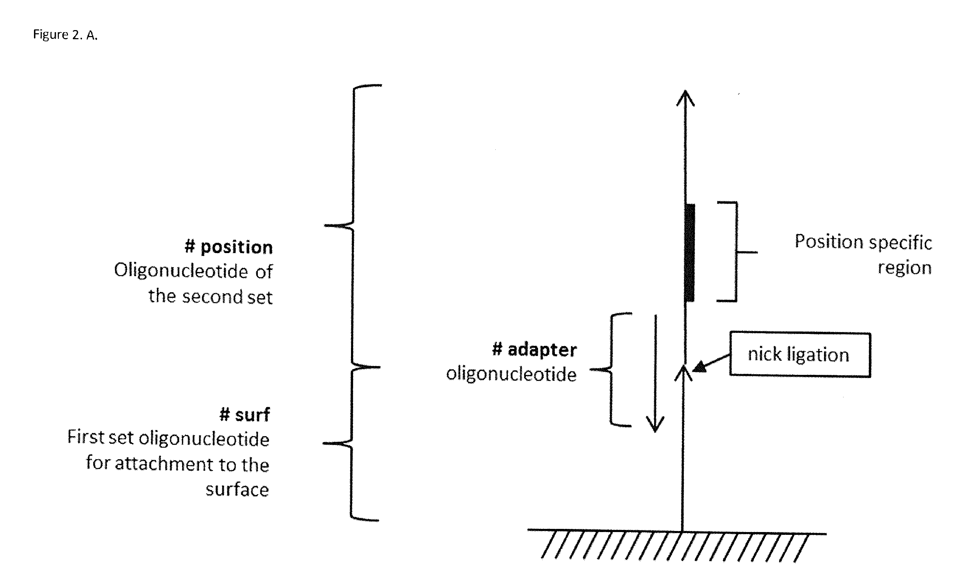

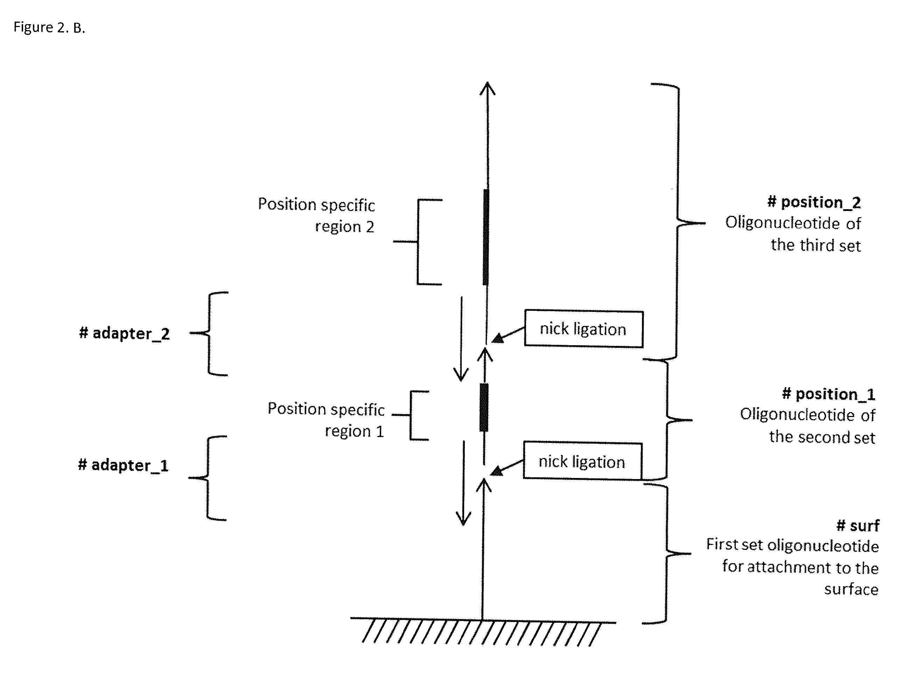

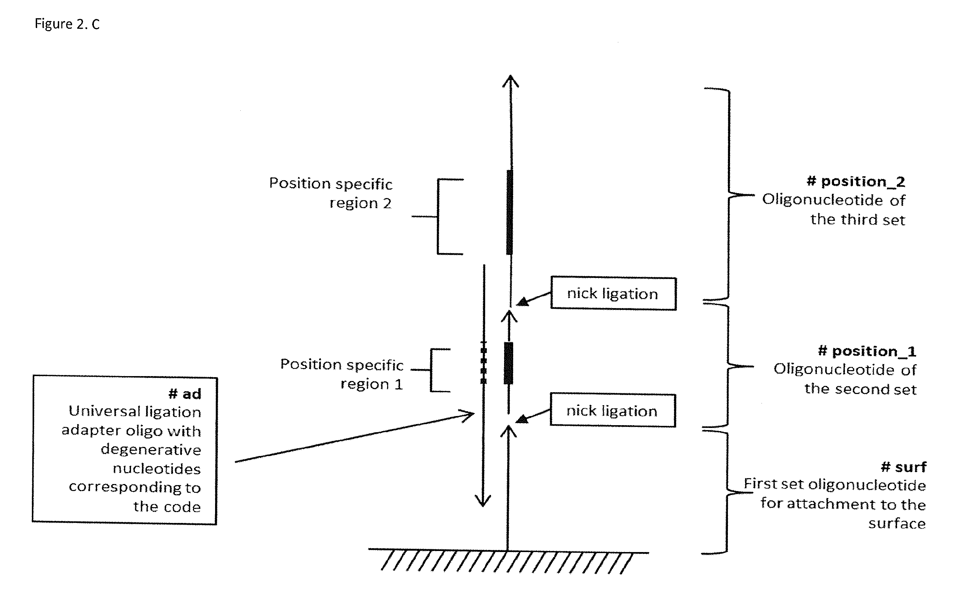

In another preferred embodiment the subsequent sets of oligonucleotides are connected using ligation, wherein the ligation results in a single stranded immobilized polynucleotide (Figure 2). In an alternate embodiment double stranded polynucleotides are used and the junction sequences in the oligonucleotides of the subsequent sets of oligonucleotides are not necessarily complementary but instead comprise a restriction site, and the connection is performed using an enzymatic digest and the ligation of the two oligonucleotides.

Depending on the selected method the two-dimensional array comprises immobilized oligonucleotides, which are single or double-stranded and comprise two label sequences. In a preferred embodiment the 2-dimensional array comprises oligonucleotides comprising two label sequences and the oligonucleotides are ordered in a manner that the labels allow an exact identification of the position of the oligonucleotide or at least a group of oligonucleotides on the array.

Depending on the method, the array comprises single or double-stranded oligonucleotides. For the preferred use of the two-dimensional array it is required, that the array comprises single-stranded oligonucleotides. Therefore if the array comprises double stranded oligonucleotides it is necessary to cleave the double-stranded oligonucleotides to receive a two-dimensional array with immobilized single-stranded oligonucleotides, which comprise at least two position-specific sequences.

The provided two-dimensional array is then suitable for further applications, for example 2d sequencing library preparation.

### Figure Legends

**Figure 1****: Examples of the extension based synthesis of full-length oligonucleotides from shorter oligonucleotides. Arrows reflect the 5'→3' direction of the oligonucleotide sequence. Dashed line corresponds to the extension reaction.**
   A. Synthesis from two oligonucleotides sets. First set of oligonucleotides is represented by oligonucleotide #surf which binds to the surface and which sequence is common for all features of the array. 3' region of #surf is a junction region required for hybridization with the second set of oligonucleotides #position. #position has a 3' region common for all features of the array and 5' position specific region. After hybridization, the site for extension reaction is formed and #surf extends along the #position, thus acquiring position-specific region. After extension and washing off the second set of oligonucleotides #position, full-length position specific oligonucleotides remain on the array.
   B. Synthesis from three oligonucleotides sets. First set of oligonucleotides is represented by oligo #surf which binds to the surface and which sequence is common for all features of the array. 3' region of #surf is a junction region required for hybridization with the second set of oligonucleotides #position_1. #position_1 has a position specific region 1, the rest of the sequence is the same for all features of the array and contain junction regions: 3' junction region is required for hybridization with #surf for hybridization and 5' junction region coincides with 3' junction region of the oligonucleotides from the third set #position_2.
      During the first extension, #surf is extended along #position_1, adding to its sequence position specific region 1 and junction region complementary to the 5' junction region of the #position_1. Then #position_1 is washed off and third set of oligonucleotides #position_2 containing position specific region 2 is added to the features of the array. The extended #surf hybridizes to the junction region of #position_2 and is extended along #position_2, adding to its sequence position specific region 2
      After washing off the third set of oligonucleotides #position_2, full-length position-specific oligonucleotides, containing two separate position-specific regions remain on the array.
   C. Synthesis from two oligonucleotides sets, where each set has a position-specific region. The principle of the scheme is the same as in A.
**Figure 2****: Ligation based synthesis of oligonucleotides**
   A. Ligation of two oligonucleotide sets. First set of oligonucleotides is represented by oligonucleotide #surf which binds to the surface and which sequence is common for all features of the array. #position represents second set of oligonucleotides and contains a position-specific region. #surf and #position are brought together by hybridization to adapter oligonucleotide #adapter and ligated, forming full-length position specific oligonucleotides, containing two separate position specific regions.
   B. Ligation of three oligonucleotide sets. Oligonucleotides of the first, second and third sets require junction sequences for hybridization to adapter sequences #adapter_1 and #adapter_2.
   C. Ligation of three oligonucleotide sets. Differs from B by using one adapter oligonucleotide. This adapter oligonucleotide #ad contains regions for hybridization to oligonucleotides of three sets. Region corresponding to the position specific sequence of the oligonucleotides of the second set is represented by polyT or polyl (inosin) sequence, which is shown as dashed line on the image. polyT or polyl (inosin) sequence is introduced to more or less the same hybrid stability for different position specific regions of oligonucleotides #position.

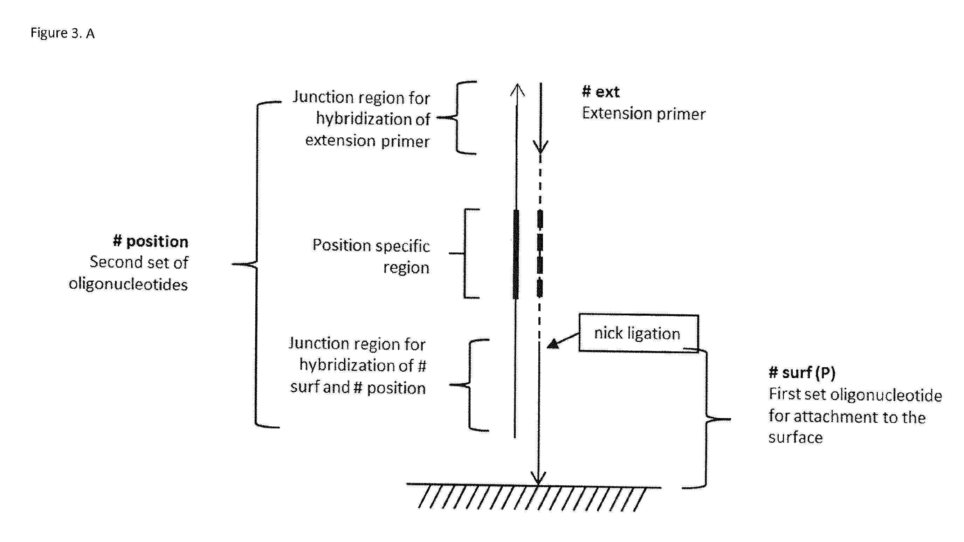

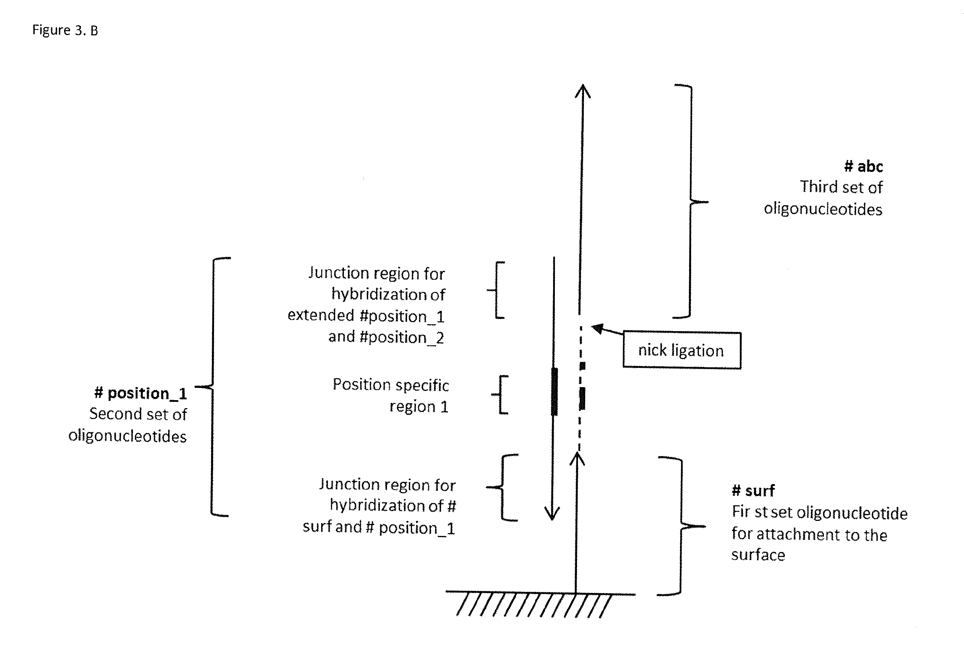

**Figure 3****: Examples of the extension-ligation based synthesis of full-length oligonucleotides from shorter oligonucleotides.**
   A. Scheme of extension-ligation based synthesis for the first set of oligonucleotides attached to the surface with 3' ends, which can't be extended. #surf (P) has a phosphate on 5' end. It is hybridized with the second set of oligonucleotides #position, which have position specific region and junction region for hybridization of oligonucleotide #ext. #ext can be extended along the #position. When it reaches the 5' end of #surf(P), the formed nick is closed with a ligase.
   B. Synthesis from three oligonucleotides sets. Second and third oligonucleotide sets are added together to the first oligonucleotide sets. Oligonucleotides #position_1 hybridize to 3' junction region of #surf and 5' junction region of #abc. The gap between hybridized oligonucleotides is filled by polymerase and the nick is closed by ligase. During extension-ligation reaction the position specific region 1 of the second set of oligonucleotides is incorporated in the full-length oligonucleotide which after washing off the #position_1 remains on the surface.

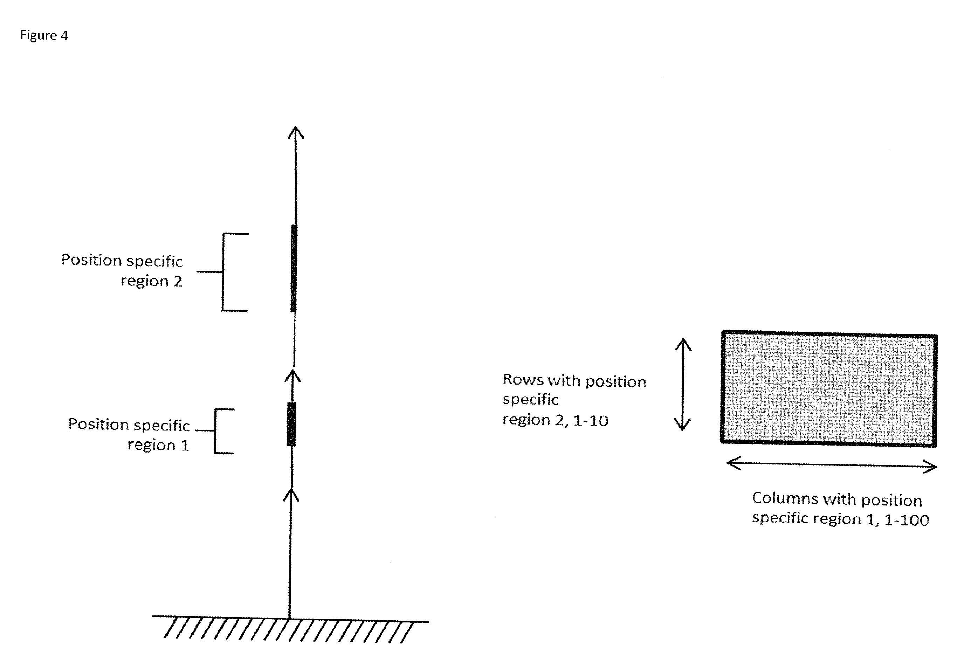

**Figure 4****: Scheme of combinatorial approach for preparation of an array with 1000 position-specific sequences using three sets of oligonucleotides.** First set of oligonucleotides contains oligonucleotides same for all features of the array. Second set of oligonucleotides with position-specific region 1 is distributed along the columns of the array, such that in each of 100 columns a specific to this column position-specific region 1 is attached to the oligonucleotides of the first set. Third set of oligonucleotides with position-specific region2 is distributed along the rows of the array, such that in each of 10 rows a specific to this row position-specific region 2 is attached to the oligonucleotides of the second set. Combination of 10 types of position-specific sequence 2 and 100 types of position-specific sequence 1 two would provide 1000 types of full-length oligonucleotides on the array. Instead of synthesizing 1000 oligonucleotides with a functional group for attachment to the surface, this scheme requires just one oligonucleotide with functional group and 120 oligonucleotides of the second and third sets. Adapter sequences (e.g. for ligation) would also be common for all features.

## Claims

1. Method for generating a two-dimensional oligonucleotide array comprising
a) providing a solid support, which allows immobilization of the first set of oligonucleotides;
b) providing a first set of synthetic oligonucleotides comprising a functional group, which allows immobilization on said solid support, optionally one or more position-labeling (sub-)sequences, optionally sequences used for the later array applications, optionally appropriate junction sequences allowing the linking of the different oligonucleotides either before or after spotting;
c) immobilizing said first set of synthetic oligonucleotides on the solid support
d) attaching to said immobilized oligonucleotides at least one additional set of oligonucleotides in an ordered manner, wherein said other sets of oligonucleotides comprise one or more position labeling (sub-)sequences, optionally appropriate junction sequences allowing the linking of the to the said first set of sequences or other sets and optionally an additional capture sequence.

2. A method according to claim 1, wherein sets of oligonucleotides are pipetted to a certain position on a microarray one after another, followed by an optional washing step.

3. A method according to claim 1, wherein the oligonucleotides of the first and/or additional sets of oligonucleotides from which the full-length oligonucleotides are synthesized have a length of less than 100 nucleotides.

4. A method according to any of the claims 2 or 3, wherein the attaching of the sets of oligonucleotides in step d) is performed using extension reaction wherein at least part of sequence(s) complementary to the oligonucleotides of next set(s) is added to the sequence of the oligonucleotides of the previous set.

5. A method according to any of the claims 1 to 3, wherein the attachment of the sets of oligonucleotides in step d) is performed by ligation, wherein ligation may be end-to end or through adapter sequences, wherein said adapter sequences are partly complementary to the junction sequences of oligonucleotides of at least two sets and wherein same or different adapter sequences may be used for ligating of subsequent sets of oligonucleotides.

6. A method according to any of the claims 1 to 3, wherein the attachment of the sets of oligonucleotides in step d) is performed by recombination, wherein oligonucleotides in the subsequent sets should have junction sequences for hybridization to each other and creating a site for recombinase.

7. A method according to any of claims 1 to 3 wherein the attachment of the sets of oligonucleotides in step d) is performed by one of the methods according to claims 4 to 6 of by a combination thereof.

8. A method according to any of the claims 1 to 7, wherein the junction sequences have a length of at least 3 nucleotides.

9. A method according to any of the claims 1 to 8, wherein the junction sequences are present in the oligonucleotides or are added to them during preparation of the array.

10. A method according to any of the claims 1 to 9 wherein the solid support is a surface made of glass, metal, plastic, nylon membrane, nitrocellulose membrane capable to or modified such that the oligonucleotides of the first set may be attached to it through the functional group, or a surface to which the oligonucleotides bind indirectly using beads attached to the surface, additional layer of a polymer, and where the surface serves just to provide the two-dimensional structure of the array.

11. A method according to any of the claims 1 to 10, wherein oligonucleotides may be DNA, RNA, PNA, LNA or a combination thereof.

12. A method according to any of the claims 1 to 10, wherein the functional group of the first set of oligonucleotides is preferably but not limited to biotin, amino -, thiol -, acrydite-modifications.

13. A method according to claims 1 to 11, wherein after step d) only oligonucleotides in close proximity comprise the same position-labeling sub-sequence(s), allowing the identification of distinct areas on said two-dimensional array.

14. A method wherein two-dimensional arrays prepared according to claims 1 to 12 are used for labeling of nucleic acid molecules applied to such array, wherein said labeling is performed by enzymatic adding of at least part of the sequence of the oligonucleotides from the array containing position-labeling region.
